# EUROPEAN PATENT APPLICATION

(11) **EP 1 978 011 A1**
(43) Date of publication of application: **08.10.2008**
(21) Application number: 06843227.7
(22) Date of filing: 26.12.2006
(51) Int. Cl.: C07C 37/70, C07C 39/16

(54) **PROCESS FOR PRODUCING BISPHENOL-A PRILL**

(30) Priority: 27.01.2006 JP 2006019790
(71) Applicant: Idemitsu Kosan Co., Ltd., Chiyoda-ku Tokyo 100-8321 (JP)
(72) Inventor: YOSHITOMI, Kazuyuki, Ichihara-shi, Chiba 2990193 (JP); MASUDA, Shuichi, Ichihara-shi, Chiba 2990193 (JP); KODAMA, Masahiro, Ichihara-shi, Chiba 2990193 (JP)
(74) Representative: Vossius & Partner
(86) International application number: PCT/JP2006/325843
(87) International publication number: WO 2007/086223

(57) **Abstract**

The present invention provides a method for regulating the phenol concentration in the circulating gas at 50 ppm by volume or less, upon producing bisphenol A prill from a bisphenol A melt using a prilling plant equipped with a prilling tower spraying and contacting the bisphenol A melt to a circulating gas, a powder remover removing bisphenol A powder from the circulating gas, a circulating gas cooler regulating the temperature of the circulating gas, and a blower making the circulating gas flow. The method avoids lowering in heat transfer performance caused by deposits formed in a heat-exchanger, increasing in pressure difference caused by deposits formed in the prilling tower or the heat-exchanger, and others in the prilling step. As a result, high-grade bisphenol A prill can be produced stably, continuously and efficiently over a long time.

## Description

### [Technical Field]

The present invention relates to a method for producing bisphenol A prill from a bisphenol A melt, more specifically, to a method for producing high-grade bisphenol A prill stably, continuously and efficiently over a long time.

### [Background Art]

Bisphenol A is used frequently as a raw material for polycarbonate that has been growing in demand in recent years as an engineering plastic. Considering handling, bisphenol A is mainly marketed in a granular solid form called as prill.
A plant for granulating prill is generally equipped with a prilling tower having at the head thereof a nozzle spraying a bisphenol A melt; a heat-exchanging unit cooling a circulating gas that is drawn out from the head of the prilling tower; a blower supplying and circulating a cooling gas; and a powder remover removing and recovering bisphenol A powder from the circulating gas that is drawn out from the head of the prilling tower. As the heat-exchanging unit, a spray tower or a heat-exchanger is used.

Among the spray prilling methods for producing bisphenol A prill described above, has been known a method in which a spray prilling tower is used and a cooling gas that is forcedly circulated is introduced into the tower in a counter-flow direction (Patent Document 1). In this case, as a heat-exchanging unit cooling the circulating gas, a cleaning tower and a heat-exchanger that cools a cleaning liquid in the tower are used.
However, when the circulating gas is cooled by using the cleaning tower, the components of the cleaning liquid is mixed in the circulating gas, thereby bringing an adverse effect to the quality of product bisphenol A. For example, when water is used as the cleaning liquid, the water content of bisphenol A is increased. This increase in the water content makes worse the fluidity of the bisphenol A, so that the bisphenol A becomes difficult to handle.
Further, another method for producing bisphenol A prill has been known, in which a bisphenol A melt is dropped through a perforated-plate placed at the upper part of the prilling tower while a cooling gas flows upward from the lower part so as to cool and solidify the resulting liquid drops (Patent Document 2).
In this Patent Document 2, a heat-exchanger (cooler) is used as the heat-exchanging unit cooling the circulating gas, and the circulating gas temperature at the outlet of the prilling tower is controlled at 60 to 80°C. However, there is no description about phenol concentration in the gas.

As described above, in a prilling tower that has a circulating system purged with an inert gas (nitrogen, for example) at a pressure somewhat higher than the atmospheric pressure, bisphenol A gas (vapor) that is vaporized from the bisphenol A melt upon forming the prill in the prilling tower, phenol gas, and impurity gases are accumulated. These accumulated gases are deposited on the surface of internal tubes or fins of a gas circulation cooling unit, bringing about lowering in the heat-exchange capacity or lowering in the flow amount of the circulating gas because these accumulated gases increases the pressure difference and work as a resistance against gas circulation. Due to this, heat-exchange between the bisphenol A melt and the circulating gas becomes insufficient in the prilling tower, and part of the bisphenol A melt that cannot be solidified into prill is deposited on the bottom of the prilling tower. The bottom is clogged with the deposits, so that the prilling tower becomes non-operable. As a result, a significant problem of discontinuation of the plant operation is brought about.
In addition, the deposits on the bottom of the prilling tower are rendered to a color scale having a high impurity concentration. The color scale is separated by any reason and is discharged out along with the bisphenol A prill. This possibly causes a product quality problem.

Patent Document 1: Japanese Patent Application Laid-Open No. 2002-534402,
Patent Document 2: Japanese Patent Laid-Open Publication No. 6-107581.

### [Disclosure of the Invention]

### [Problems to be Solved by the Invention]

Under the circumstances described above, upon producing bisphenol A prill from a bisphenol A melt, it is an object of the present invention to provide a method for producing high-grade bisphenol A prill stably, continuously and efficiently over a long time by avoiding lowering in heat transfer performance, increasing in pressure difference, and the like caused by deposits formed in the heat-exchanger in the prilling step.

### [Means for Solving the Problems]

The present inventors have made intensive studies to address the problems described above. As a result, the present inventors have found that the bisphenol A melt obtained in an adduct decomposition step discharges gas such as phenol, bisphenol A, and isopropenyl phenol from the surface of the melt drops when these drops are solidified into prill; the gas is accumulated in the prilling tower; and cyclic dimmers formed from bisphenol A or isopropenyl phenol are deposited, with the help of phenol that serves as a binder, on the inside of the prilling tower or on the surfaces of the tubes or fins of a gas circulation cooling unit that have a lot of cooling surface. The present inventors have also found that high-grade bisphenol A prill can be produced stably, continuously and efficiently over a long time by regulating the phenol concentration in the circulating gas in the prilling tower. Based on this finding, the present invention has been accomplished.

Namely, the present invention provides the following method for producing bisphenol A prill.
1. A method for producing bisphenol A prill including regulating phenol concentration in a circulating gas at 50 ppm by volume or less, upon producing bisphenol A prill from a bisphenol A melt using a prilling plant equipped with a prilling tower spraying and contacting the bisphenol A melt to the circulating gas, a powder remover removing bisphenol A powder from the circulating gas, a circulating gas cooler regulating the temperature of the circulating gas, and a blower making the circulating gas flow.
2. The method for producing bisphenol A prill as described in (1), wherein the circulating gas is an inert gas.
3. The method for producing bisphenol A prill as described in (1) or (2), wherein a part of the circulating gas is drawn out of the prilling system while fresh inert gas is supplied to the prilling system in an amount of 60 Nm³ or more per 1 ton of the bisphenol A melt supplied to the prilling plant, so that the phenol concentration in the circulating gas is controlled at 50 ppm by volume or less.

4. The method for producing bisphenol A prill as described in any of (1) to (3), wherein the temperature of a cooling water that is used as a cooling medium for the circulating gas cooler is controlled at 20 to 35°C.
5. The method for producing bisphenol A prill as described in any of (1) to (4), wherein the circulating gas cooler is a multi-tube heat-exchanger having fins, and a tube bundle of the multi-tube heat-exchanger is detachable on a local site.
6. The method for producing bisphenol A prill as described in any of (1) to (5), wherein the bisphenol A melt is produced through the following steps: (A) a condensation reaction step in which acetone and an excess amount of phenol are condensed in the presence of an acidic catalyst; (B) a concentration step in which the reaction mixture obtained in the condensation reaction step is concentrated; (C) a crystallization and solid/liquid separation step in which the concentrated liquid obtained in the concentration step is cooled so as to crystallize an adduct of bisphenol A and phenol, and separate the concentrated liquid into the adduct and a mother liquid; and (D) an adduct decomposition step in which phenol is removed from the adduct of the bisphenol A and phenol so as to recover bisphenol A, after that (E) a bisphenol A melt obtained in the adduct decomposition step is sprayed into prill.

### [Effect of the Invention]

In the method for producing bisphenol A prill according to the present invention, upon producing bisphenol A prill from a bisphenol A melt, it can be avoided lowering in heat transfer performance caused by deposits formed in a heat-exchanger and increase in pressure difference caused by deposits formed in a prilling tower or the heat-exchanger in a prilling step. Thereby, high-grade bisphenol A prill can be stably, continuously and efficiently produced over a long time.

### [Brief Description of Drawings]

FIG 1 is an explanatory drawing illustrating an example of a flow sequence of a prilling plant in the method for producing bisphenol A prill according to the present invention.
FIG 2 is an explanatory drawing illustrating a structure of a vertical-type multi-tube heat-exchanger (with fins) used for a circulating gas cooler.

### [Explanation of Reference Numerals]

1: Flow channel for supplying bisphenol A melt,
2: Nozzle chamber,
3: Prilling tower,
4: Circulating gas flow channel,
5: Powder remover (Bag filter),
6: Flow channel for discharging bisphenol A powder,
7: Blower (Circulating gas booster),
8: Circulating gas cooler,
9: Bisphenol A prill,
10: Nozzle plate (Perforated plate),
11: Crane for lifting tube bundle of heat-exchanger,
12: Hook for lifting tube bundle of heat-exchanger,
13: Tube bundle of multi-tube heat-exchanger (with fins),
14: Circulating gas flow channel,
15: Cooling water flow channel, and
16: Outer body of heat-exchanger (Casing).

### [Best Mode for Carrying Out the Invention]

Bisphenol A production in which the method for producing bisphenol A prill according to the present invention is applied includes the following steps of (A) a condensation reaction step, (B) a concentration step, (C) a crystallization and sold/liquid separation step, (D) an adduct decomposition step, and (E) a prilling step. Hereinafter, each step will be explained in detail.

### (A) Condensation reaction step

In the condensation reaction step, phenol and acetone are reacted in the presence of an acidic catalyst. Source phenol and acetone are reacted in a manner that phenol is in excess stoichiometrically. The molar ratio of phenol/acetone is usually in the range of from 3 to 30 and preferably from 5 to 20. The reaction temperature is usually 50 to 100°C. The reaction pressure is usually normal pressure to 1.5 MPa and preferably normal pressure to 0.6 MPa. The catalyst includes usually a strongly acidic cation-exchange resin such as a sulfonic acid type. May further be included a catalyst that is given by neutralizing a part of the strongly acidic cation-exchange resin with an auxiliary catalyst such as mercapto alkylamine. For example, may be included a catalyst whose sulfonic acid group is neutralized by about 5 to 30 mol% with 2-mercapto ethylamine, 3-mercapto propylamine, N,N-dimethyl-3-mercapto propylamine, N,N-di-n-butyl-4-mercapto butylamine, 2,2-dimethyl thiazolidine, and the like. Reaction between phenol and acetone is carried out in at fixed-bed flow process that is a continuous plug-flow process or in a suspension-bed batch process. In the case of the fixed-bed flow process, the liquid hourly space velocity (LHSV) of a source liquid introduced into a reactor is about 0.2 to 50 hr⁻¹. In the case of the suspension-bed batch-wise process, the amount of a resin catalyst is in the range of generally from 20 to 100% by mass with respect to the source liquid and the reaction time is about 0.5 to about 5 hours, although they depend on the reaction temperature and pressure.

### (B) Concentration step

In the concentration step, the reaction mixture obtained in the condensation reaction step is concentrated. The reaction mixture from the condensation reaction step is usually concentrated in two-steps. In a first concentration step, unreacted acetone, reaction product water, and others are removed by vacuum distillation or the like. Vacuum distillation is carried out usually at a temperature of 30 to 180°C under a pressure of 13 to 67 kPa. Then, in a second concentration step, phenol is distilled out so as to control the bisphenol A concentration. At this time the bisphenol A concentration is adjusted at preferably 20 to 60% by mass. At a bisphenol A concentration of 20% by mass or higher, yield becomes increased. On the other hand, at a bisphenol A concentration of 60% by mass or lower, solidification temperature is lowered, thereby enabling easy transportation. Therefore, usually in the first concentration step, the reaction mixture liquid is concentrated in advance and adjusted at a concentration within the above range. Usually it is desirable to carry out the second concentration under a pressure of 4 to 40 kPa and at a temperature of 70 to 140°C.

### (C) Crystallization and solid/liquid separation step

In the crystallization and solid/liquid separation step, the concentrated liquid obtained in the concentration step is cooled so as to crystallize an adduct of bisphenol A and phenol and to separate the concentrated liquid into the adduct and a mother liquid. The concentrated liquid from the concentration step is usually cooled from a temperature of 70 to 140°C to a temperature of 35 to 60°C, so that an adduct of bisphenol A and phenol is crystallized and that the concentrated liquid is changed into a slurry liquid. The concentrated liquid is cooled by an external heat-exchanger or by way of heat remove by vaporization of water that is added to a crystallizer.
After that, the slurry liquid is subjected to solid/liquid separation. The mother liquid obtained in this crystallization and solid/liquid separation step contains reaction water, so that it is usually introduced into a dehydration tower. Note that, a part of the mother solution that contains water may be circulated back to the crystallizer. The composition of the mother liquid obtained after dehydration is usually as: phenol is 65 to 85 % by mass; bisphenol A is 10 to 20% by mass; and by-products such as 2,4'-isomers are 5 to 15% by mass. The mother liquid contains a large amount of impurities such as 2,4'-isomers. This mother liquid is treated in the following isomerization step so as to recover phenol and bisphenol A from the mother liquid.

The adduct recovered by the solid/liquid separation is forwarded to an adduct decomposition step in which phenol is removed to obtain high-purity bisphenol A. A solid that contains mainly the adduct and is deposited on the surface of a filter of the solid/liquid separator after filtration is subjected to washing with a cleaning liquid. The cleaning liquid may include phenol that is recovered by evaporation, source phenol, water, and a water-phenol mixed liquid, and may also include a solution similar to a bisphenol A saturated phenol solution. The much the amount of the cleaning liquid used the better of course, from the viewpoint of cleaning efficiency. However, the amount has an own upper limit considering the dissolution loss of crystals and the circulation, recovery and reuse of the cleaning liquid. The amount considered to be most efficient is usually about 0.1 time to 10 times of the crystals on a mass basis. Note that, the crystals may be re-dissolved after the crystallization and solid/liquid separation, and the crystallization and solid/liquid separation may be repeated. The impurities incorporated in the adduct crystals are decreased successively by repeating the crystallization and solid/liquid separation in multi-stages.
In this occasion, as a liquid used for the re-dissolution and a cleaning liquid used to wash the solid that is obtained by solid/liquid separation and contains mainly the adduct, phenol that is recovered by evaporation, source phenol, water, and a water-phenol mixed liquid, and also a solution similar to a bisphenol A saturated phenol solution may be used in each stage. In addition, the mother liquid obtained by re-crystallization and solid/liquid separation may be recycled to the crystallization step in the former stage.
The solid/liquid separator used for the solid/liquid separation is not particularly limited as long as the separator is the one that is usually used, but examples of the separator may include a belt filter, a drum filter, a tray filter, a centrifugal separator, and the like.

### (D) Adduct decomposition step

In the adduct decomposition step, phenol is removed from the adduct of bisphenol A and phenol so as to recover bisphenol A. In the adduct decomposition step, phenol is removed from the adduct that is recovered by solid/liquid separation, so that high-purity bisphenol A is obtained.
Namely, the adduct is heated and fused at a temperature of about 100 to 160 to decompose it into bisphenol A and phenol. Most of the phenol is removed from the resulting melt with a distillation drum or the like, and the remaining phenol is further removed by steam stripping, so that a melt of bisphenol A is obtained.

### (E) Prilling step

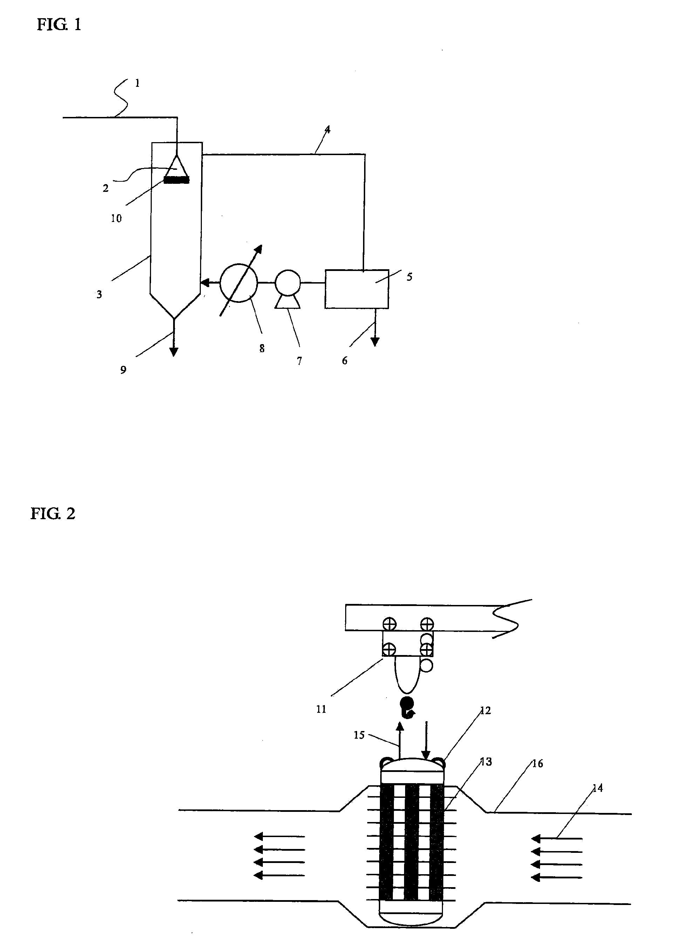

In the prilling step, the bisphenol A melt obtained in the adduct decomposition step is granulated so as to separate a product bisphenol A. FIG 1 shows an example of a flow sequence of a prilling plant in the method for producing bisphenol A prill according to the present invention.
In FIG 1, the bisphenol A melt obtained in the adduct decomposition step is forwarded from a flow channel 1 to a head of a prilling tower 3, and then sprayed through a number of holes of a nozzle plate 10 placed in a nozzle plate chamber 2 installed at the head of the tower. The sprayed melt is cooled with a circulating gas 4 flowing upward from the bottom of the prilling tower 3 and is drawn out through a flow channel 9 as granular solids called as prill, providing product bisphenol A prill.
The circulating gas 4 discharged from the head of the prilling tower is accompanied by bisphenol A powder. The accompanied powder is collected and recovered with a powder remover 5 such as a bag filter. The recovered powder may be drawn out of the prilling system through a flow channel 6 or may be fused and directly returned back to the bisphenol A production process. The circulating gas which is removed the powder is pressurized with a blower 7 and is introduced from the bottom of the prilling tower and circulated again after the gas temperature is conditioned with a circulating gas cooler 8.
Hereinafter will be explained each unit used in the prilling step.

### (1) Prilling tower

In the prilling tower, the bisphenol A melt obtained in the adduct decomposition step is supplied to a nozzle plate through an orifice and a strainer in the nozzle plate chamber. The hole size of the nozzle plate is from 0.3 to 1.0 mm. The rate of bisphenol A melt flowing through the nozzle is preferably from 0.5 to 1.8 m/s, and the flow rate of the circulating gas is preferably from 0.7 to 2.0 m/s. Further, the spacing between the holes is preferably from 5 to 12 mm. By passing through the orifice, the flow to each nozzle can be made uniform. With the help of the strainer having an aperture smaller than the hole size, clogging of nozzle plate holes caused by foreign substances can be avoided.
The nozzle plate placed at the head of the tower is installed preferably at a recessed position in the prilling tower so that the spraying from the nozzle holes is not influenced directly by the flow of the circulating gas.
The nozzle plate desirably has a slide gate valve, so that the nozzles are allowed to be replaced during operation.
At the head of the prilling tower, plural spray plates are installed. The spray plate placed at the outmost periphery preferably has a holding distance from the inside wall or the prilling tower, so that the bisphenol A liquid drops sprayed from the spray plate are not deposited on the inside wall of the prilling tower.
The plural nozzle plates are preferably arrayed uniformly (in two lines or circular, for example) in an area keeping the holding distance satisfying the above condition from the inside wall of the prilling tower. Owing to this configuration, can be attained an uniform heat exchange between bisphenol A melt sprayed from the nozzle plate and the circulating gas, thereby reducing the opportunity of depositing the melt on the bottom of the tower before solidification.
At the bottom of the prilling tower, a uniformly divided aperture serving as an inlet of the circulating gas and a detachable current plate are equipped, so that a uniform flow of the circulating gas can be attained. Further, the corn-shaped inside surface of the bottom of the tower is desirably finished with a buff #200 or more and a knocker is equipped on the wall surface of the bottom so as to make bisphenol A blocks deposited on the bottom easily drop off.

### (2) Powder remover

Examples of a typical powder remover may include a bag filter. Bisphenol A powder is a substance extremely sticky and easily bridge-building. Due to this, not only a knocker is equipped on the wall surface of the bag filter, but also the inside surface thereof is buff-finished (#200 or more) and a blaster discharging a large amount of an inert gas at a time into the inside is equipped desirably.
Note that, inert gas such as nitrogen gas is usually used to remove and recover the bisphenol A powder deposited on the bag filter, but in the present invention, the amount of the inert gas used in the bag filter does not meet the amount of the inert gas that is supplied to regulate the phenol concentration in the circulating gas. Therefore, besides the inert gas used in the bag filter, additional fresh inert gas is needed to be supplied in the prilling system so as to regulate the phenol concentration in the circulating gas at 50 ppm by volume or less.

### (3) Circulating gas cooler

As the circulating gas cooler, a heat-exchanger that is conventionally used to cool gas is used. A multi-tube heat-exchanger composed of circular tubes with fins is preferably equipped. For example, as shown in FIG 2, a vertical-type multi-tube heat-exchanger (with fins) is used. Preferably the heat-exchanger has a tube bundle that can be detached and/or attached on a local site, for example, by lifting the tube bundle with a crane.
FIG 2 shows a crane 11 for lifting a tube bundle of a heat-exchanger; a hook 12 for lifting the tube bundle of the heat-exchanger; a tube bundle 13 of a multi-tube heat-exchanger (with fins); a circulating gas flow channel 14; a cooling water flow channel 15; and an external body of the heat-exchanger (casing) 16.
The deposits adhered to the detached tubes and fins of the heat-exchanger can be removed by washing them with solvent (acetone, phenol, or the like, for example).
Note that, deposition of impurities or the like on metal surface can be prevented by regulating the temperature of the cooling water that is used as a cooling medium for the circulating gas cooler at 20 to 35°C.

In the present invention, an inert gas can be used as the circulating gas. There is not any particular limitation on the inert gas, but usually nitrogen gas is used.
The present invention is characterized by regulating the phenol concentration in the circulating gas in the prilling step at 50 ppm by volume or less and preferably at 30 ppm by volume or less. By regulating at 50 ppm by volume or less, deposition of Bisphenol A powder or impurities on the circulating gas cooler or the inside of the prilling tower can be avoided. Due to this, the amount of the circulating gas is not lowered by the increase of the pressure difference in the gas circulating system, thereby allowing a continuous operation over a long time. In addition, can also be prevented effectively the formation of deposits (scale) on the heat-exchanger tubes of the gas circulation cooler, so that the circulating gas can be cooled efficiently.
Here, the phenol concentration in the circulating gas can be measured with a portable gas detector (Kitagawa precision gas detector, for example).

Examples of a method for regulating the phenol concentration in the circulating gas at 50 ppm by volume or less in the prilling step may include: (1) a method in which a part of the circulating gas is drawn out of the prilling system while a fresh inert gas is supplied into the prilling system; (2) a method in which a part of the circulating gas is passed through an absorption unit so as to absorb the phenol contained in the circulating gas in active carbon or the like, and then returned back to the prilling system; (3) a method in which a part of the circulating gas is passed through a gas cleaning unit so as to absorb the phenol contained in the circulating gas in pure water, and then returned back to the prilling system; and the like.
In the method (1), preferably a fresh inert gas is supplied to the prilling system in an amount of 60 Nm³ or more per 1 ton of the bisphenol A melt that is supplied to the prilling plant.
In the methods (1) to (3), there is not any particular limitation on the position at which the gas after phenol absorption or cleaning or the inert gas is supplied (or retuned back) into the prilling system and the position at which the part of the circulating gas is drawn out. However, when the gas after phenol absorption or cleaning or the inert gas is introduced into an intake line of the blower shown in FIG 1 while the part of the circulating gas is drawn out through an outlet line of the circulating gas cooler, effects on the environment can be reduced in the method (1), and the absorption and cleaning can be performed advantageously in the methods (2) and (3), that is, the blower is no longer needed. In the method (3), a sprinkler tower or a packed tower serves as the gas cleaning unit, and is desirably dried by heating or with a dehydrator after gas cleaning.

### [Examples]

Hereinafter, the present invention will be described in detail with reference to the following examples, but it should be construed that the present invention is in no way limited to those examples.

### Example 1

In the flow sequence shown in FIG 1, a bisphenol A melt was fed to the head of the prilling tower at a rate of 10 t/h so as to obtain prill while a circulating gas (70,000 Nm³/hr) was pressurized with the blower (outlet pressure: 3 kPa-G) and supplied from the bottom of the tower.
Nitrogen gas was supplied to the prilling tower at a rate of 1,500 Nm³/h (150 Nm³/t-bisphenol A melt) and was discharged from the prilling tower at the same rate so as to control under a slightly pressurized condition (1kPa-G) and to control the phenol concentration in the circulating cooling gas at 10 to 30 ppm by volume. The temperature of the cooling water passed through the circulating gas cooler (multi-tube heat-exchanger with fins having a heat-exchanging surface area of 3,000 m²) was controlled at 20°C to 35°C with a cooling water tower.
By regulating the operation conditions as described above, the inlet temperature of the circulating gas cooler was controlled at 67 to 68°C throughout a year. Thereby, the outlet temperature of the heat-exchanger for the circulating gas was able to be controlled at an objective temperature of 40°C during the operation. In addition, in the course of the continuous operation over a year, only a small increase of about 5 kPa in the pressure difference was brought about in the heat-exchanger. Decrease in the gas flow rate caused by this pressure difference increase was not experienced. Further, decrease in the heat removal capability due to the lowering in the overall heat transfer coefficient caused by the deposits on the fins was not experienced. And faint yellow color scale was discharged at a rate of about 0.01 kg/day from a vibrating screen placed at the bottom of the prilling tower.

### Example 2

Similarly to Example 1, a bisphenol A melt was fed to the head of the prilling tower at a rate of 10 t/h so as to obtain prill while a circulating cooling gas (70,000 Nm³/hr) was pressurized with the blower (outlet pressure: 3 kPa-G) and supplied from the bottom of the tower.
Nitrogen gas was supplied to the prilling tower at a rate of 700 Nm³/h (70 Nm³/t-bisphenol A melt) and was discharged from the prilling tower at the same rate so as to control under a slightly pressurized condition (1 kPa-G) and to regulated the phenol concentration in the circulating cooling gas at 20 to 50 ppm by volume. The temperature of the cooling water passed through the circulating gas cooler (multi-tube heat-exchanger with fins having a heat-exchanging surface area of 3,000 m²) was controlled at 20°C to 35°C with a cooling water tower.
By regulating the operation conditions as described above, the inlet temperature of the circulating gas cooler was kept at 67 to 68°C throughout a year. Thereby, the outlet temperature of the heat-exchanger for the circulating gas was able to be controlled at an objective temperature of 40°C during the operation. In addition, in the course of the continuous operation over a year, only a small increase of about 8 kPa in the pressure difference was brought about in the heat-exchanger. Decrease in the gas flow rate caused by this pressure difference increase was not experienced. Further, decrease in the heat removal capability due to the lowering in the overall heat transfer coefficient caused by the deposits on the fins was not experienced. And faint yellow color scale was discharged at a rate of about 0.02 kg/day from a vibrating screen placed at the bottom of the prilling tower.

### Comparative Example 1

Similarly to Example 1, a bisphenol A melt was fed to the head of the prilling tower at a rate of 10 t/h so as to obtain prill while a circulating cooling gas (70,000 Nm³/hr) was pressurized with the blower (outlet pressure: 3 kPa-G) and supplied from the bottom of the tower.
Nitrogen gas was supplied to the prilling tower at a rate of 300 Nm³/h (30 Nm³/t-bisphenol A melt) and was discharged from the prilling system at the same rate so as to control under a slightly pressurized condition (1 kPa-G) and to regulated the phenol concentration in the circulating cooling gas at 60 to 120 ppm by volume. The temperature of the cooling water passed through the circulating gas cooler (multi-tube heat-exchanger with fins having a heat-exchanging surface area of 3,000 m²) was controlled at 20°C to 35°C with a cooling water tower.
As a result, during 3 months from the start of the operation, the inlet temperature of the circulating gas cooler was kept at 67 to 68°C, and the outlet temperature of the heat-exchanger for the circulating gas was able to be controlled at a temperature of 40°C. After that period, however, the pressure difference in the circulating gas cooler increased and the gas flow rate was decreased. Further, deposits were formed on the fins and the overall heat transfer coefficient was also decreased. Due to this, the outlet temperature of the circulating gas cooler became no longer kept at the objective 40°C, and the outlet temperature was gradually increased. Thus, the heat-exchanging ability between the circulating gas and the sprayed bisphenol A melt was decreased in the prilling tower. After 4 months from the start of the operation, bisphenol A that was not allowed to be solidified in the prilling tower frequently deposited and formed blocks at the bottom of the tower. Eventually, the plant operation was stopped and the circulating gas cooler (multi-tube heat-exchanger with fins) was cleaned up. Further, scale that was separated from product prill and was colored heavily in dark yellow was discharged at a rate of about 1 kg/day from a vibrating screen placed at the bottom of the prilling tower.

### [Industrial Applicability]

According to the present invention, upon producing bisphenol A prill from a bisphenol A melt, lowering in heat transfer performance and increasing in pressure difference caused by deposits formed in a heat-exchanger in a prilling step can be avoided, thereby producing high-grade bisphenol A prill stably, continuously and efficiently over a long time.

## Claims

1. A method for producing bisphenol A prill comprising:
regulating phenol concentration in a circulating gas at 50 ppm by volume or less upon producing bisphenol A prill from a bisphenol A melt using a prilling plant equipped with a prilling tower spraying and contacting the bisphenol A melt to the circulating gas, a powder remover removing bisphenol A powder from the circulating gas, a circulating gas cooler regulating the temperature of the circulating gas, and a blower making the circulating gas flow.

2. The method for producing bisphenol A prill according to claim 1, wherein the circulating gas is an inert gas.

3. The method for producing bisphenol A prill according to claim 1 or 2, wherein
a part of the circulating gas is drawn out of the prilling system while fresh inert gas is supplied to the prilling system in an amount of 60 Nm³ or more per 1 ton of the bisphenol A melt supplied to the prilling plant, so that the phenol concentration in the circulating gas is regulated at 50 ppm by volume or less.

4. The method for producing bisphenol A prill according to any of claims 1 to 3, wherein
the temperature of a cooling water that is used as a cooling medium for the circulating gas cooler is controlled at 20 to 35°C.

5. The method for producing bisphenol A prill according to any of claims 1 to 4, wherein
the circulating gas cooler is a multi-tube heat-exchanger having fins, and a tube bundle of the multi-tube heat-exchanger is detachable on a local site.

6. The method for producing bisphenol A prill according to any of claims 1 to 5, wherein
the bisphenol A melt is produced through the following steps: (A) a condensation reaction step in which acetone and an excess amount of phenol are reacted in the presence of an acidic catalyst; (B) a concentration step in which the reaction mixture obtained in the condensation reaction step is concentrated; (C) a crystallization and solid/liquid separation step in which the concentrated liquid obtained in the concentration step is cooled so as to crystallize an adduct of bisphenol A and phenol, and separate the concentrated liquid into the adduct and a mother liquid; and (D) an adduct decomposition step in which phenol is removed from the adduct of the bisphenol A and phenol so as to recover bisphenol A, after that (E) a bisphenol A melt obtained in the adduct decomposition step is sprayed into prill.
